# EUROPEAN PATENT SPECIFICATION

(11) **EP 4 713 316 B1**
(45) Date of publication and mention of the grant of the patent: **24.06.2026**
(21) Application number: 24809358.5
(22) Date of filing: 20.11.2024
(51) Int. Cl.: C07D 251/24, A61K 8/49, A61Q 17/04

(54) **A PROCESS FOR INCREASING THE CONTENT OF 2,4-BIS[4-(2-ETHYLHEXYLOXY)-2-HYDROXYPHENOL]-6-(4-METHOXYPHENOL)-1,3,5-TRIAZINE (BEMT) IN A COMPOSITION COMPRISING BEMT**
VERFAHREN ZUR ERHÖHUNG DES GEHALTS AN 2,4-BIS[4-(2-ETHYLHEXYLOXY)-2-HYDROXYPHENOL)-6-(4-METHOXYPHENOL)-1,3,5-TRIAZIN (BEMT) IN EINER ZUSAMMENSETZUNG MIT BEMT
PROCÉDÉ POUR AUGMENTER LA TENEUR EN 2,4-BIS-[4-(2-ETHYLHEXYLOXY)-2-HYDROXYPHÉNOL!-6-(4-METHOXYPHENOL)-1,3,5-TRIAZINE (BEMT) DANS UNE COMPOSITION COMPRENANT BEMT

(30) Priority: 30.11.2023 EP 23213458
(43) Date of publication of application: 25.03.2026
(60) Divisional application: 26179257.6
(73) Proprietor: BASF SE, 67056 Ludwigshafen am Rhein (DE)
(72) Inventor: KRUCK, Matthias, 79639 Grenzach-Wyhlen (DE); CHENG, Chung Yuan, Kaohsiung City, Taiwan, 83162 (TW); KRONEMAYER, Helmut, 67056 Ludwigshafen am Rhein (DE); KIEDORF, Gregor, 67056 Ludwigshafen am Rhein (DE); KRIESTEN, Martin, 67056 Ludwigshafen am Rhein (DE)
(74) Representative: BASF IP Association
(86) International application number: PCT/EP2024/082963
(87) International publication number: WO 2025/114106

(56) References cited:
- WO-A1-03/075875
- JP-A- 2011 213 635
- NONE: "Verfahren zur Veretherung von 2,4-Bis(2,4-dihydroxyphenyl)-(4methoxyphenyl)-1,3,5-triazin zu 2,4-Bis[4-(2-ethylhexyloxy)-2hydroxyphenyl]-6-(4-methoxyphenyl)-1,3,5-triazin (BEMT) An IP.com Prior Art Database Technical Disclosure", 29 May 2019 (2019-05-29), pages 1 - 6, XP093152176, Retrieved from the Internet <URL:https://ip.com/IPCOM/000258609> [retrieved on 20240416]
- OLIVEIRA DIOGO NOIN DE ET AL: "In vitroevaluation of Sun Protection Factor and stability of commercial sunscreens using mass spectrometry", JOURNAL OF CHROMATOGRAPHY B, ELSEVIER, AMSTERDAM, NL, vol. 988, 19 February 2015 (2015-02-19), pages 13 - 19, XP029149525, ISSN: 1570-0232, DOI: 10.1016/J.JCHROMB.2015.02.018

## Description

### Technical field

The present invention relates to an improved process for the preparation of 2,4-bis[4-(2-ethylhexyloxy)-2-hydroxyphenol]-6-(4-methoxyphenol)-1,3,5-triazine (BEMT, CAS 187393-00-6), especially to a process producing a composition having increased content of BEMT. The present invention further relates to said composition of BEMT comprising less impurities as usually obtained in the synthesis of BEMT. The present invention further relates to the use of said composition for protection of human and animal hair and skin from the damaging effects of UV radiation.

Hence, the present invention also relates to a cosmetic preparation comprising said composition.

### Background

Bis-resorcinyl triazines are highly effective UV-absorbers which may, for example, be used as light stabilizers in plastics or as intermediates in the preparation of alkyl substituted bis-resorcinyl triazine derivatives such as for example Tinosorb^{®}S which are particularly suitable as light screening agents in cosmetic products. The preparation of bis-resorcinyl triazines is known and e.g., disclosed in US 5,955,060. The preparation encompasses the reaction of cyanuric chloride with a phenyl magnesium bromide compound in a Grignard reaction to a dichlorotriazine. The two resorcinyl groups are then introduced by a Friedel-Crafts acylation with resorcinol in the presence of a Lewis acid, in particular an aluminium halide. In a third step, the etherification of the free 4-hydroxyl groups is carried out by alkylation such as known from US 5,955,060.

However, the former preparation method has the disadvantage of being complicated and has low yields. Furthermore, the Friedel-Crafts acylation often yields unwanted by-products which are hardly removable and are subsequently carried over to the product.

Hence, WO 2016/184764 A1 describes a process for the preparation of bis-resorcinyl triazines which is easy to carry out and affords economic and regulatory advantages because of higher yields and higher purities.

Furthermore, the process described in US 5,955,060 is not satisfactory in view of reaction times and selectivity. Even more, this process involves chromatography techniques to isolate the products, which are tedious, labor intensive and time consuming and thus not suitable for industrial scale as this leads to unacceptable manufacturing cost.

Hence, WO 2016/184766 A1 describes a process for the preparation of bis-resorcinyl triazines of formula (I) involving specific bases and reaction conditions, which is useful for scale up and results in high yields and purities.

The processes as described, however, have the disadvantages that the solid-liquid separation and purification of the resulting crystals suffers from long filtration and washing times, due to small particles sizes obtained during crystallization. This further induces a lower degree in purification.

CN 113929636 A1 describes a purification process of bis-ethyl hexyloxyphenol methoxyphenyl triazine combining silica gel column chromatography with C6 alkane recrystallization. However, this process lacks the technical relevance in industrial multi-ton processes due to the chromatography step involved.

IP.com 2017 17(8A) and IP.com 2019 19(6) describe certain processes to produce 2,4-bis[4-(2-ethylhexyloxy)-2-hydroxyphenol]-6-(4-methoxyphenol)-1,3,5-triazine (BEMT) involving crystallization steps from various solvents improving the purity of the BEMT produced. Nevertheless, the problem of long filtration and washing times still remains.

### Summary of the invention

Hence, it is a first object of the present invention to find a process for the production of 2,4-bis[4-(2-ethylhexyloxy)-2-hydroxyphenol]-6-(4-methoxyphenol)-1,3,5-triazine (BEMT) being more economically feasible, in particular involving less time preferably during the filtration and washing steps and producing high yields and purities at the same time. It is a further object of the present invention to prepare a composition comprising BEMT allowing for faster post synthesis processing at high purity levels.

It has now surprisingly found out that above-mentioned objects can be achieved by a process for increasing the content of 2,4-bis[4-(2-ethylhexyloxy)-2-hydroxyphenol]-6-(4-methoxyphenol)-1,3,5-triazine (BEMT) in a composition comprising BEMT, the process comprising the steps of:
a) providing a composition comprising BEMT and impurities;
b) dissolving said composition in a solvent mixture at a first dissolving temperature yielding an unsaturated BEMT solution, wherein the solvent mixture comprises at least one polar solvent;
c) decreasing the temperature of the unsaturated BEMT solution to a saturation temperature, wherein the saturation temperature is lower than the first dissolving temperature, yielding a saturated BEMT solution;
d) further decreasing the temperature of the saturated BEMT solution to a first crystallization temperature, wherein the first crystallization temperature is lower than the saturation temperature, thereby yielding a supersaturated BEMT solution;
e) adding seed crystals of **BEMT** in an amount in the range of from 0.00001 wt.-% to 20 wt.-% with respect to the total weight of said composition to the supersaturated **BEMT** solution during step d) yielding a first **BEMT** suspension comprising **BEMT** particles;
f) heating the first **BEMT** suspension to a second dissolving temperature yielding a second **BEMT** suspension comprising less **BEMT** particles than the first **BEMT** suspension, wherein the second dissolving temperature is higher than the first crystallization temperature and lower than the first dissolving temperature;
g) decreasing the temperature of the second **BEMT** suspension to a second crystallization temperature yielding a final **BEMT** suspension comprising larger **BEMT** particles than the first BEMT suspension;
h) isolating the BEMT particles from the final BEMT suspension,
   wherein the second crystallization temperature is lower than the first crystallization temperature.

It has been further surprisingly found out that the above-mentioned objects can be achieved by a particulate composition comprising at least 98.0 wt.% 2,4-bis[4-(2-ethylhexyloxy)-2-hydroxyphenol]-6-(4-methoxyphenol)-1,3,5-triazine (BEMT), based on the total weight of the composition and at least one of, preferably at least two of, more preferably at least three of, even more preferably at least four of, still even more preferably at least five of, still even more preferably at least six of, still even more preferably at least seven of, and most preferably all of the compounds according to formulae (I) to (VIII): 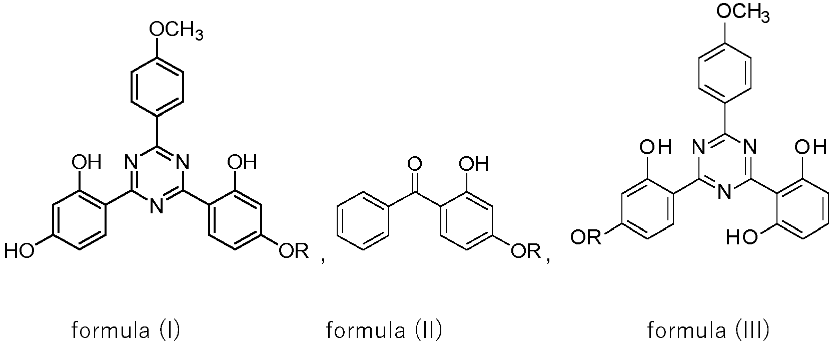 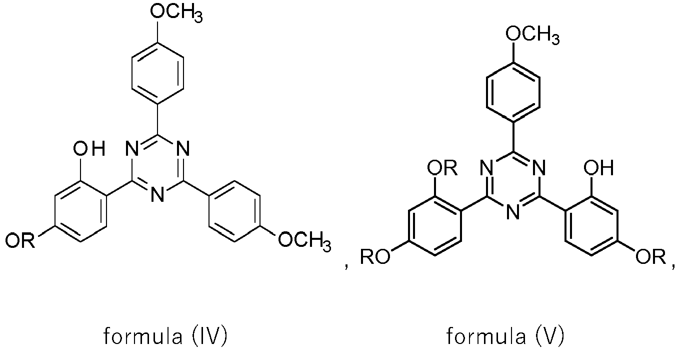 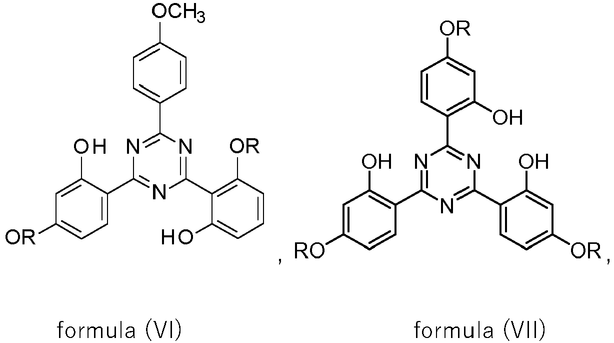 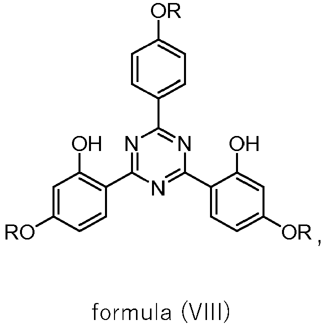 wherein
R is a 2-ethylhexyl residue;
the amount of the compound of formula (I) in the composition is less than 0.06 wt.-%, preferably less than 0.05 wt.-%, and most preferably less than 0.03 wt.-%, based on the total weight of the composition;
the amount of the compound of formula (II) in the composition is less than 0.11 wt.-%, preferably less than 0.05 wt.-%, more preferably less than 0.03 wt.-%, based on the total weight of the composition;
the amount of the compound of formula (III) in the composition is less than 0.11 wt.- %, preferably less than 0.07 wt.-%, and most preferably less than 0.05 wt.-%, based on the total weight of the composition;
the amount of the compound of formula (IV) in the composition is less than 0.41 wt.- %, preferably less than 0.23 wt.-%, and most preferably less than 0.12 wt.-%, based on the total weight of the composition;
the amount of the compound of formula (V) in the composition is less than 0.14 wt.-%, preferably less than 0.11 wt.-%, and most preferably less than 0.03 wt.-%, based on the total weight of the composition;
the amount of the compound of formula (VI) in the composition is less than 0.07 wt.- %, preferably less than 0.03 wt.-%, based on the total weight of the composition;
the amount of the compound of formula (VII) in the composition is less than 0.05 wt.- %, preferably less than 0.03 wt.-%, based on the total weight of the composition;
the amount of the compound of formula (VIII) in the composition is less than 0.17 wt.- %, preferably less than 0.15 wt.-%, based on the total weight of the composition; and the particulate composition has a median particle size D_{50,3} of more than 10 µm,
preferably equal to or more than 12 µm, determined according to ISO 13320:2020.

For a person skilled in the art, it is obvious that the above-mentioned process also reduces the amount of any other impurity potentially present in crude BEMT mixtures that are not specifically mentioned.

More preferably, the particulate composition of the present invention comprises at least one of, preferably at least two of, more preferably at least three of, and most preferably all of the compounds according to formulae (IX) to (XII): 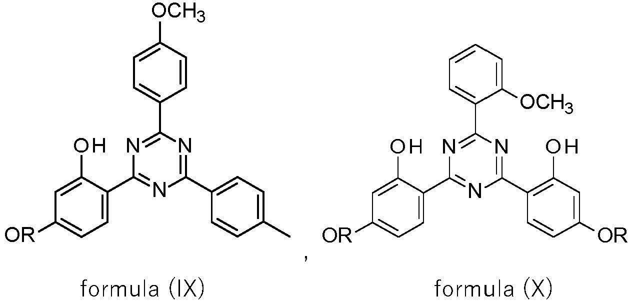 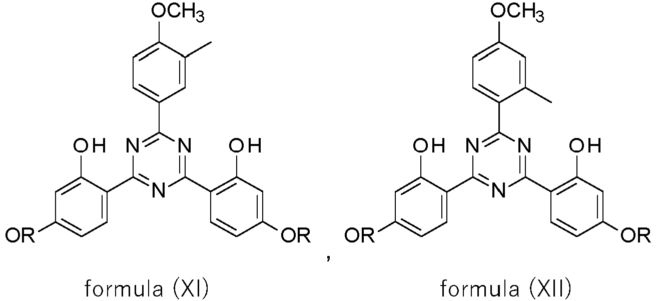 wherein
R is a 2-ethylhexyl residue;
The amount of the compound of formula (IX) in the composition is less than 0.05 wt.- %, preferably less than 0.03 wt.-%, based on the total weight of the composition;
the amount of the compound of formula (X) in the composition is less than 0.05 wt.-%, preferably less than 0.03 wt.-%, based on the total weight of the composition;
the amount of the compound of formula (XI) in the composition is less than 0.05 wt.-% based on the total weight of the composition; and
the amount of the compound of formula (XII) in the composition is less than 0.05 wt.- % based on the total weight of the composition.

It has been further found that the above-mentioned objects can be achieved by a use of the particulate composition according to the present invention for protection of human and animal hair and skin from the damaging effects of UV radiation. Furthermore, said objects can be also achieved by a cosmetic preparation comprising the particulate composition according to present invention with cosmetically compatible carriers and auxiliaries.

The present invention is especially advantageous as the crystallization methods allows for controlled growth of bigger crystals leading to a reduced filter resistance of the filter cake in the filtration and washing steps. Furthermore, the controlled crystal growth has the beneficial effect of producing a powder having a larger average particle size and an extraordinary purity in particular in view of side-products of the synthesis method used.

### Brief Description of the Drawings

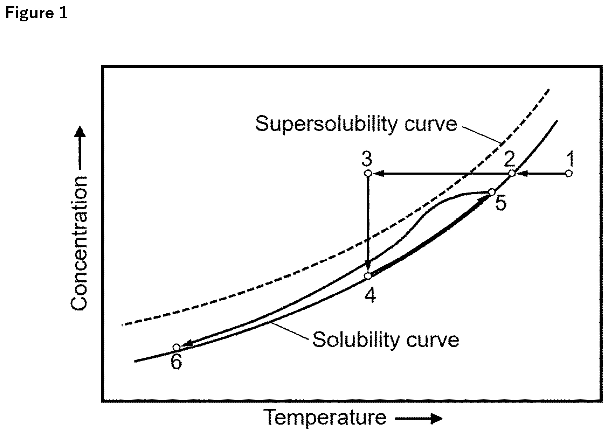

- Figure 1: is a schematic illustration of the process of the invention according to a preferred embodiment in a solubility curve diagram.

### Reference signs

- 1: Represents steps a) to b)
- 2: Represents step c)
- 3: Represents steps d) and e)
- 4: Represents a preferable end of steps d) and e)
- 5: Represents step f)
- 6: Represents step g)

### Definitions

Before describing in detail exemplary embodiments of the present invention, definitions which are important for understanding the present invention are given.

The term *"impurity"* or *"impurities"* as used herein denotes a material within a composition, which has a molecular structure different from the material, which forms the highest amount of the composition.

The term *"standard pressure"* as used herein denotes a pressure in the range of 1,012 to 1,014 mbar, preferably 1,013.25 mbar.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±10 %, preferably ±8 %, more preferably ±5 %, even more preferably ±2 %. It is to be understood that the term "comprising" and "encompassing" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only and is not intended to limit the scope of the present invention that will be limited only by the appended claims. Unless defined otherwise, all technical and scientific terms used herein have the same meanings as commonly understood by one of ordinary skill in the art.

As used herein the term "does not comprise", "does not contain", or "free of" means in the context that the composition of the present invention is free of a specific compound or group of compounds, which may be combined under a collective term, that the composition does not comprise said compound or group of compounds in an amount of more than 0.8 % by weight, based on the total weight of the composition. Furthermore, it is preferred that the composition according to the present invention does not comprise said compounds or group of compounds in an amount of more than 0.5 % by weight, preferably the composition does not comprise said compounds or group of compounds at all.

When referring to compositions and the weight percent of the therein comprised ingredients it is to be understood that according to the present invention the overall amount of ingredients does not exceed 100% (± 1% due to rounding).

### Detailed Description of the Invention

In the following the process of the invention and the product of the invention are described in detail.

### Process of the invention

As described above, the process of the present invention is a process for increasing the content of 2,4-bis[4-(2-ethylhexyloxy)-2-hydroxyphenol]-6-(4-methoxyphenol)-1,3,5-triazine (BEMT) in a composition comprising BEMT, the process comprising the steps of:
a) providing a composition comprising BEMT and impurities;
b) dissolving said composition in a solvent mixture at a first dissolving temperature yielding an unsaturated BEMT solution, wherein the solvent mixture comprises at least one polar solvent;
c) decreasing the temperature of the unsaturated BEMT solution to a saturation temperature, wherein the saturation temperature is lower than the first dissolving temperature, yielding a saturated BEMT solution;
d) further decreasing the temperature of the saturated BEMT solution to a first crystallization temperature, wherein the first crystallization temperature is lower than the saturation temperature, thereby yielding a supersaturated BEMT solution;
e) adding seed crystals of BEMT in an amount in the range of from 0.00001 wt.-% to 20 wt.-% with respect to the total weight of said composition to the supersaturated BEMT solution during step d) yielding a first BEMT suspension comprising BEMT particles;
f) heating the first BEMT suspension to a second dissolving temperature yielding a second BEMT suspension comprising less BEMT particles than the first BEMT suspension, wherein the second dissolving temperature is higher than the first crystallization temperature and lower than the first dissolving temperature;
g) decreasing the temperature of the second BEMT suspension to a second crystallization temperature yielding a final BEMT suspension comprising larger BEMT particles than the first BEMT suspension;
h) isolating the BEMT particles from the final BEMT suspension,
   wherein the second crystallization temperature is lower than the first crystallization temperature.

The process of the invention yields the particulate compositions comprising at least 98 wt.% 2,4-bis[4-(2-ethylhexyloxy)-2-hydroxyphenol]-6-(4-methoxyphenol)-1,3,5-triazine (BEMT), based on the total weight of the composition, of the present invention. The BEMT particles obtained in step h) of the inventive process represent the particulate composition of the invention.

### Step a)

Preferably, the step a) comprises the step of synthesizing a composition comprising BEMT. Preferably, the step of synthesizing a composition comprising BEMT comprises the step of reacting 2,4-Bis(2,4-dihydroxyphenyl)-(4-methoxyphenyl)-1,3,5-triazine in dimethylformamide with a 2-ethylhexyl halide R-X, wherein X is CI, Br or I and R is a 2-ethylhexyl group, in the presence of a base selected from the group consisting of sodium carbonate, sodium phosphate and sodium hydrogencarbonate, and the reaction temperature is selected in the range of at least 120° C (at atmospheric pressure).

It is well understood that the reaction temperature would have to be adjusted accordingly if pressure/vacuum would be applied to the process of the present invention, which temperature, however, could easily be adjusted by a person skilled in the art and which embodiment is incorporated herein as well.

Dimethylformamide [CAS 68-12-2] is also known as N,N-Dimethylformamide. Suitable 2-ethylhexyl halides encompass in particular the respective bromide or chloride, the chloride being preferred. Most preferably 2-ethylhexyl chloride [CAS 123-04-6] is used in the processes according to the present invention.

Sodium carbonate [CAS 497-19-8], sodium phosphate [CAS 7601 -54-9] and sodium hydrogen carbonate [CAS 144-55-8] are well known to a person skilled in the art and can e.g. be purchased at Sigma-Aldrich. In all embodiments of the present invention, preferably anhydrous sodium carbonate or anhydrous sodium hydrogen carbonate, most preferably anhydrous sodium carbonate is used. Most preferably anhydrous sodium carbonate with a purity > 99%, such as preferably with a purity of > 99.5%, most preferably with a purity of > 99.9% (assay, calculated based on dry substance) is used in the step of synthesizing a composition comprising BEMT of step a) of the process of the present invention.

The reaction temperature (at atmospheric pressure, i.e. 1013 mbar) is preferably selected in the range of 120-155° C, more preferable in the range of 130-155° C, and most preferably in the range of 130-145° C. In a very advantageous embodiment, the reaction temperature is selected such that the reaction is maintained at reflux (i.e. a reaction temperature of about 133-143° C, at atmospheric pressure), which can easily be adjusted by a person skilled in the art. If desired the reaction could also be performed under reduced or increased pressure, while the temperature is adjusted accordingly, which is well known by a person skilled in the art. Preferably the reaction is, however, carried out at atmospheric pressure. The reaction time generally adjusted such that all of the 2,4-bis(2,4-dihydroxyphenyl)-(4-methoxyphenyl)-1,3,5-triazine is consumed and the amount of mono-alkylated product is < 8 %, preferably < 5 %, more preferably < 4% (traced by HPLC: area %, detection at 230 nm). Advantageously, the reaction time is selected in the range of 3 to 24 h, preferably in the range of 4 to 20h, most preferably in the range of 5 - 15 h.

The molar ratio of the base to the 2,4-bis(2,4-dihydroxyphenyl)-(4-methoxyphenyl)-1,3,5-triazine is preferably selected in the range of 2 to 9, most preferably in the range of 3 to 7. The amount of dimethylformamide is preferably selected such that the amount of the 2,4-bis(2,4-dihydroxyphenyl)-(4-methoxyphenyl)-1,3,5-triazine in dimethylformamide is in the range of 0.5 to 2 mol/l, preferably in the range of 0.75 to 1.5 mol/l, most preferably in the range of 0.8 to 1 mol/l.

Preferably, the 2-ethylhexyl halide R-X is used in a slight excess. More preferably, the amount of the 2-ethylhexyl halide R-X is selected in the range of 1.5 to 6 mol-equivalents, even more preferably in the range of 2 to 5 mol-equivalents, still even more preferably in the range of 2.5 to 4 mol-equivalents, and most preferably in the range of 3 to 3.5 mol-equivalents relative to the 2,4-Bis(2,4-dihydroxyphenyl)-(4-methoxyphenyl)-1,3,5-triazine. Preferably, the base is added in two portions, the first portion being used to neutralize the solution of the 2,4-bis(2,4-dihydroxyphenyl)-(4-methoxyphenyl)-1,3,5-triazine in dimethylformamide to about pH 6.5 to 7.5, preferably to about pH 7. Generally, about 15-30 wt.-% based on the total amount of the base is to be used for the neutralization step, the amount being easily adjustable by a person skilled in the art.

Thus, in a particular advantageous embodiment the step of synthesizing in step a) according to the present invention encompasses the following consecutive steps, wherein all the definitions and preferences as given above also apply:
(i) suspending the 2,4-bis(2,4-dihydroxyphenyl)-(4-methoxyphenyl)-1,3,5-triazine in dimethylformamide
(ii) heating the resulting suspension to 90-155° C, preferably to 95-145° C,
(iii) neutralization of the resulting suspension to about pH 7 with the first portion of the base,
(iv) addition of the second portion of the base, followed by the addition of the 2-ethylhexyl halide R-X
(v) heating of the resulting mixture to reflux.

Advantageously, the reaction time in step (v) is selected in the range of 3 to 24h, preferably in the range of 4 to 20h, most preferably in the range of 5 -15h. In a further advantageous embodiment, the step of synthesizing a composition comprising BEMT of step a) of the process of the present invention encompasses the further steps of
(vi) filtration of the resulting reaction mixture obtained in step (v)
(vii) neutralization of the reaction mixture obtained in step (vi) using sodium bisulfate and removal of volatile solvents under ambient or reduced pressure.

### Step b)

In the process according to the present invention, the at least one polar solvent is preferably a polar organic solvent, more preferably is selected from the group consisting of methanol, ethanol, 1-butanol, 2-butanol, isopropyl alcohol, acetone, methyl ethyl ketone, diethyl ketone, N,N-dimethyl formamide, and most preferable is acetone. Preferably, the solvent mixture comprises only one polar solvent, preferably consists of the polar solvent. More preferably, the solvent mixture consists of a solvent selected from the group consisting of methanol, ethanol, 1-butanol, 2-butanol, isopropyl alcohol, acetone, methyl ethyl ketone, diethyl ketone, N,N-dimethyl formamide, most preferably the solvent mixture consists of acetone. Acetone assures the best solvent system with respect to crystallization, used temperature and controllability of the crystallization process. Furthermore, acetone is easily available and cheap. Finally, acetone has a low boiling point and good volatility to allow efficient removal of acetone from the BEMT crystals.

Preferably, the unsaturated BEMT solution of step b) is a solution comprising 10 to 60 wt.- % BEMT, more preferably 20 to 40 wt.-%, and most preferably 25 to 35 wt.-%.

Preferably, in the process according to the present invention the first dissolving temperature is higher than the clearing temperature resulting from the solubility curve for the solvent mixture and BEMT at standard pressure and lower than the boiling temperature of the solvent mixture at standard pressure. Hence, the temperature of the unsaturated BEMT solution yielded in step b) can be higher than necessary to solve the amount of BEMT implemented in the amount of solvent mixture implemented. However, more preferably, the first dissolving temperature is the clearing temperature resulting from the solubility curve for the solvent mixture and the BEMT at standard pressure. As the unsaturated BEMT solution is further cooled down to eventually reach a supersaturated state, a first dissolving temperature higher than the clearing temperature resulting from the solubility curve for the solvent mixture and the BEMT at standard pressure requires unnecessary cooling time and energy. Furthermore, if the first dissolving temperature is equal or higher than the boiling temperature of the solvent mixture at standard pressure, unnecessarily high amounts of energy are used rendering the process less energy efficient. It should be further understood that the first dissolving temperature is not the lowest temperature allowing for any BEMT to be solved in the solvent mixture at standard pressure, otherwise a cooling is not possible anymore. Hence, preferably, the first dissolving temperature is higher than a temperature, which is X K higher than the lowest temperature allowing for any BEMT to be solved in the solvent mixture at standard pressure, wherein X is 15% of the temperature difference between the lowest temperature allowing for any BEMT to be solved in the solvent mixture at standard pressure and the boiling temperature of the solvent mixture at standard pressure, preferably 50%, more preferably 70% and most preferably 80%.

Preferably, step b) comprises a final step of removing, preferably filtrating, undissolved solids from the saturated BEMT solution.

### Steps c), d), and e)

BEMT is generally obtained as a mixture of three stereoisomers, i.e.:

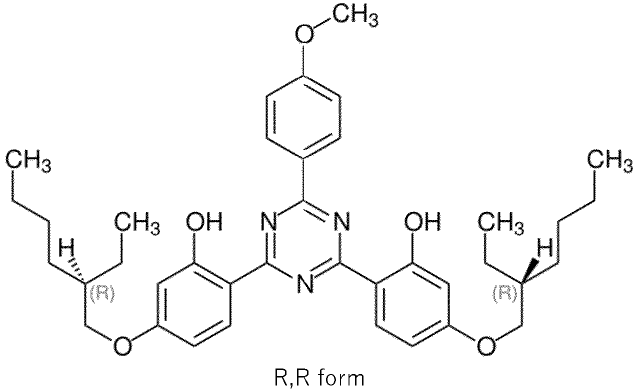

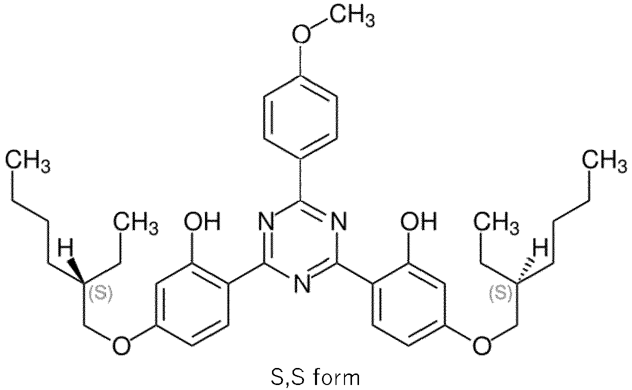

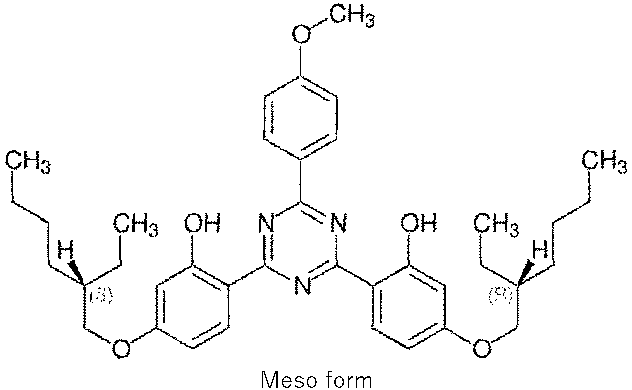

These three different stereoisomers are difficult to separate and therefore are usually not separated at all. Generally, due to the presence of these three different stereoisomers, BEMT tends to have especially in polar organic solvents such as acetone hindered nucleation and growth kinetics as regards crystallization. Hence, it is generally a problem to crystallize BEMT from a solution. Thus, both, bringing an unsaturated solution of BEMT into a highly supersaturated state by cooling the solution down to a first crystallization temperature and seeding with BEMT crystals at the same time is usually used to force **BEMT** solutions to crystallize. However, such heavy approach leads to high nucleation rates (for nucleation, in general, "(···) a free-energy barrier must be passed in order to form [molecule] clusters of a critical size, beyond which the new phase grows spontaneously" (Mersmann, A. (Ed.) (2001) Crystallization Technology Handbook (2nd ed.))). leading to large number of very fine crystals and respectively small particle sizes having an impact on the purity of the BEMT composition and filtration efficiency.

The present invention introduces a seeding loop via steps d), e), and f), wherein a supersaturated solution is formed and seeded with seed crystals (cf. references 2, 3, and 4 of Figure 1). Subsequently, the temperature is raised again (cf. references 4 and 5 of Figure 1). As the dissolution of BEMT is normally not kinetically hindered, the system follows more or less the solubility curve up to the point representing the adjusted temperature. This step ensures that the number of crystallization centers in the BEMT suspension is significantly reduced. After a second cooling step, the suspension is controlled to fully crystallize.

The saturation temperature of step c) is preferably at or close to the point on the solubility curve for the given BEMT/solvent mixture system (cf. reference 2 in Figure 1).

As in step d) of the process according to the present invention a supersaturated BEMT solution is formed, the first crystallization temperature is preferably lower than the clearing temperature resulting from the solubility curve for the solvent mixture and the BEMT at standard pressure.

The process according to any one of the preceding claims, wherein in step e) the seed crystals are added at least once at a temperature below, preferably in a range of from 1 to 15 K below, the saturation temperature of the supersaturated BEMT solution, preferably in a range of from 1 to 5 K below the saturation temperature of the supersaturated BEMT solution. Preferably, the polar solvent is acetone, and the seeding crystals are added at a temperature in the range of from 35 to 20 ° C, preferably 30 to 20 ° C, most preferably 25 to 20 ° C. If the polar solvent is acetone and the seeding crystals are added batchwise, the batchwise addition preferably is carried out at 23 ° C, 22 ° C, and 21 ° C.

Hence, preferably, in the process according to the present invention, in step e), the seed crystals of BEMT are added in one batch, in two batches at two distinct times, in three batches at three distinct times, in four batches at four distinct times, or in up to ten batches at ten distinct times. Most preferably, in the process according to the present invention, in step e), the seed crystals of BEMT are added in one batch.

Preferably, the amount of seed crystals of BEMT added in total in step e) is not higher than 15 wt.-%, more preferably not higher than 10 wt.-%, and most preferably not higher than 5 wt.-%. Moreover, preferably, the amount of seed crystals of BEMT added in total in step e) is at least 0.00005 wt.-%, more preferably at least 0.0001 wt.-%, and most preferably at least 0.0005 wt.-%.

In a preferred embodiment of the process of the present invention, in step e) the temperature of the supersaturated BEMT solution is kept constant at the first crystallization temperature for at least 30 min. The effect of this waiting time is that larger amounts of small BEMT particles are formed.

### Step f)

It should be noted that the first BEMT suspension of step f) can be a supersaturated or a saturated BEMT suspension. A supersaturated BEMT suspension is a suspension of BEMT particles in a supersaturated BEMT solution. As a supersaturated BEMT solution is kinetically hindered, it relaxes over the time thereby BEMT is crystallizing. As soon as the supersaturated BEMT suspension reaches its thermodynamical equilibrium, it becomes a saturated BEMT suspension (cf. references 3 to 4 in Figure 1). Preferably, the first BEMT suspension of step f) is a saturated BEMT suspension, assuring that step f) is carried out at the solubility curve thereby achieving the lowest number of crystallization centers. Preferably, in the process according to the present invention, the second dissolving temperature of step f) is higher than the maximum temperature the first BEMT suspension could reach by uptake of the released crystallization enthalpy. This ensures that a higher number of crystallization centers is dissolved again, and a lower number of crystallization centers remains in the suspension.

### Step g)

In another preferred embodiment of the process according to the present invention in step g) the second BEMT suspension is kept constantly at the second crystallization temperature for at least 30 min. This waiting time has the effect that the crystallization is completed, and the yield is maximized.

Preferably, step g) of the process of the present invention is carried out stepwise at two different cooling rates, preferably at three cooling rates. Thereby, preferably, the cooling rates are kept constant for a certain period of time, thereby forming cooling ramps with a constant cooling rate. Preferably, the first cooling rate is lower than the second cooling rate. Also preferably, the second cooling rate is lower than the third cooling rate. This assures that the crystallization follows a pattern contrary to the natural pattern i.e., in that in the beginning of step g) lower amounts of BEMT crystallize and in the end of step g) higher amounts of BEMT crystallize. More preferably, the polar solvent is acetone, and the first cooling rate is in the range of from -0.5 to -4 K/h, preferably in the range of from -0.7 to - 1.5 K/h and most preferably is -1.2 K/h. Also more preferably, the polar solvent is acetone, and the second cooling rate is in the range of from -1 to -6 K/h, preferably in the range of from -2 to -4 K/h, and most preferably is -3 K/h. Also more preferably, the polar solvent is acetone, and the third cooling rate is in the range of from -3 to -10 K/h, preferably in the range of from -4 to -8 K/h, and most preferably is -5.3 K/h. Also preferably, the polar solvent is acetone, and the time of the first cooling ramp is in the range of from 2 to 8 h, further preferably, the polar solvent is acetone, and the time of the second cooling ramp is in the range of from 1 to 5 h, further preferably, the polar solvent is acetone, and the time of the third cooling ramp is in the range of from 1 to 5 h, this further enhances the growth of lower numbers of crystallization center and reduces the forming of new nuclei. Hence, the resulting crystal size is increased.

When comparing the cooling of the respective unsaturated BEMT solution of step d) and the second BEMT suspension of step g) it is preferred that the maximum value of any shortest distance of any point of the graph, which the unsaturated BEMT solution follows in the concentration/temperature diagram, to the solubility curve of the BEMT/solvent mixture system, is higher than the maximum value of any shortest distance of any point of the graph, which the second BEMT suspension follows in the concentration/temperature diagram, to the solubility curve of the BEMT/solvent mixture system. This ensures that the crystallization force induced by the cooling of the respective solution or suspension is higher in step d) and lower in step g). Therefore, in step d) more nuclei are formed than in step g). It should be noted that due to the presence of the seeding step e) this feature is not an essential feature of the present invention.

Furthermore, when comparing the cooling of steps d) and g) it is preferred that the overall cooling rate of step d) is higher (i.e., faster) than the overall cooling rate of step g). Even more preferred, the cooling rate of step d) is higher (i.e., faster) than any of the first, second, or third cooling rates of step g).

In the process according to the present invention the second crystallization temperature of step g) is lower than the first crystallization temperature assuring higher yields of BEMT crystals. Preferably, the polar solvent is acetone, and the second crystallization is lower than 10 ° C, more preferably the polar solvent is acetone, and the second crystallization temperature is 0 ° C.

### Step h)

Preferably, step h) comprises a step of solid-liquid separation to isolate the BEMT particles from the final BEMT suspension, preferably by filtration. Also preferably, step h) comprises the step of washing the BEMT particles, preferably with a polar solvent. More preferably, step h) comprises a step of solid-liquid separation to isolate the BEMT particles from the final BEMT suspension, preferably by filtration followed by washing the isolated BEMT particles with a suitable polar solvent. Preferably, the polar solvent is acetone.

### Other steps

In a further preferred embodiment according to the present invention, the process comprises a further step i) of heating the BEMT particles to further remove the solvent mixture.

Preferably, the process according to the present invention comprises one or more repetitions of steps b) to h). Repetition of these steps even further reduces the amounts of the compounds according to formulae (I) to (XII) in the final BEMT composition. More preferably, the process according to the present invention comprises not more than one repetition. Selecting only one repetition achieves the best ratio of required energy and time to the reduction of amounts of the compounds according to formulae (I) to (XII).

### Particulate composition of the present invention

The present invention further relates to a particulate composition comprising at least 98 wt.% 2,4-bis[4-(2-ethylhexyloxy)-2-hydroxyphenol]-6-(4-methoxyphenol)-1,3,5-triazine (BEMT), based on the total weight of the composition and at least one of, preferably at least two of, more preferably at least three of, even more preferably at least four of, still even more preferably at least five of, still even more preferably at least six of, still even more preferably at least seven of, and most preferably all of the compounds according to formulae (I) to (VIII), wherein R is a 2-ethylhexyl residue; the amount of the compound of formula (I) in the composition is less than 0.06 wt.-%, preferably less than 0.05 wt.-%, and most preferably less than 0.03 wt.-%, based on the total weight of the composition; the amount of the compound of formula (II) in the composition is less than 0.11 wt.-%, preferably less than 0.05 wt.-%, more preferably less than 0.03 wt.-%, based on the total weight of the composition; the amount of the compound of formula (III) in the composition is less than 0.11 wt.-%, preferably less than 0.07 wt.-%, and most preferably less than 0.05 wt.-%, based on the total weight of the composition; the amount of the compound of formula (IV) in the composition is less than 0.41 wt.-%, preferably less than 0.23 wt.-%, and most preferably less than 0.12 wt.-%, based on the total weight of the composition; the amount of the compound of formula (V) in the composition is less than 0.14 wt.-%, preferably less than 0.11 wt.-%, and most preferably less than 0.03 wt.-%, based on the total weight of the composition; the amount of the compound of formula (VI) in the composition is less than 0.07 wt.-%, preferably less than 0.03 wt.-%, based on the total weight of the composition; the amount of the compound of formula (VII) in the composition is less than 0.05 wt.-%, preferably less than 0.03 wt.-%, based on the total weight of the composition; the amount of the compound of formula (VIII) in the composition is less than 0.17 wt.-%, preferably less than 0.15 wt.-%, based on the total weight of the composition; and the particulate composition has a median particle size D_{50,3} of more than 10 µm, preferably equal to or more than 12 µm, determined according to ISO 13320:2020. The amounts of the components in wt.% are determined according to the invention via HPLC according to USP monograph proposal Vol. 32(4), 1045-1047, 2006.

When referring to the total weight of the composition, it is directly apparent that this refers to the total weight of the particulate composition of the present invention.

In a preferred embodiment of the particulate composition of the present invention the particulate composition comprises at least one of, preferably at least two of, more preferably at least three of, and most preferably all of the compounds according to formulae (XI) to (XII), wherein R is a 2-ethylhexyl residue; the amount of the compound of formula (IX) in the composition is less than 0.05 wt.-% based on the total weight of the composition; the amount of the compound of formula (X) in the composition is less than 0.05 wt.-% based on the total weight of the composition; the amount of the compound of formula (XI) in the composition is less than 0.05 wt.-% based on the total weight of the composition; and the amount of the compound of formula (XII) in the composition is less than 0.05 wt.-% based on the total weight of the composition.

Preferably, in the particulate composition according to the present invention the amount of BEMT in the composition is more than 98 wt.-% with respect to the total weight of the composition, preferably more than 99 wt.-%.

The median particle size D_{50,3} of the particulate composition according to the present invention is more than 10 µm, preferably equal to or more than 12 µm determined by laser diffraction according to ISO 13320:2020 as described herein in the section *'measurement methods'.* The larger the particle size of the produced particulate composition is, the lower the filter cake resistance in the filtration and washing process resulting in lower filtration and washing times.

### Other embodiments

Another embodiment of the present invention is the use of the particulate composition according to the present invention for protection of human and animal hair and skin from the damaging effects of UV radiation.

Hence, the present invention also relates to a cosmetic preparation comprising the particulate composition according to present invention with cosmetically compatible carriers and auxiliaries.

Preferably, the cosmetic preparation according to the present invention comprises other UV protection substances, preferably selected from the group consisting of triazines, oxanilides, triazoles, amides containing vinyl groups and cinnamic acid amides.

### Examples

### Measurement methods

### a) Weight percentages in composition

The composition of BEMT samples was determined via HPLC according to USP monograph proposal Vol. 32(4), 1045-1047, 2006 by use of internal standards to obtain wt.% values. Relative retention times of impurities were confirmed via synthesis. Identification of further impurities was achieved via coupled methods like HPLC-MS. The side-products of formula (I) to formula (VIII) are listed as NK1 to NK8 in Table 1 below.

### b) Particle sizes and distribution

The volume-weighted particle size density distributions of the particles inside the final BEMT suspensions were determined by laser diffraction according to ISO 13320:2020 on a Malvern Mastersizer 3000, equipped with a Malvern Hydro SM wet sample dispersion unit and additional cooling to temper the final BEMT suspension samples at 0° C. The Fraunhofer approximation was chosen as optical model. The obtained particle size is defined according to ISO 13320:2020 as the equivalent spherical diameter. The median particle diameter D_{50,3} is defined as in ISO 13320:2020. It is to be understood that the terms "particle size" and "particle diameter" are synonyms.

### c) Filter cake resistance

The filter cake resistance of the final BEMT suspensions was determined according to the guideline VDI 2762 Part 2:2010-12 (R2016). The resulting values are presented as the mathematical product of the filter cake resistance and the viscosity in units of mPas/m².

### Experiments

### Reference Example 1 (RE1)

In this example, crude BEMT has been produced. All steps were carried out using protective atmosphere (nitrogen). A reactor equipped with a condenser and a water separator was charged with 54 g sodium carbonate and 260 g N,N-dimethyl formamide. The reaction mixture was heated to 80° C and agitated. 126 g 2,4-Bis(2,4-dihydroxyphenyl)-(4-methoxyphenyl)-1,3,5-triazine (Assay 80%, >5% NaCl, <1% water) were added at once. Subsequently, 156 g 2-ethylhexyl chloride were added dropwise and the reactor temperature increased to first 90° C, then 120° C. Finally, the reaction temperature was increased to first 140 ° C, then 155° C, while a mixture of water, 2-ethylhexyl chloride, DMF and other compounds was distilled off. Phases of the distillate were collected and separated. The organic phase was constantly fed back to the reactor. Reaction progress was monitored via HPLC and alkylation stopped at maximum yield. The reaction mixture was allowed to cool down to 120 ° C and was filtered. The filtrate (F1) was cooled down to 90 ° C and neutralized by addition of aqueous sodium bisulfate. Subsequently, solvents were removed in vacuo to yield crude BEMT. The obtained crude BEMT melt contained about 90 wt.-% BEMT (measured by calibrated HPLC) and specified side-products in amounts according to table 1. The remainder consisted of solvents and non-specified by-products.

### Comparative Example 1 (CE1)

The crude BEMT melt obtained in RE1 was dissolved in acetone, the solution filtered and heated to 40° C to yield a 29% solution of the crude BEMT in acetone. The mixture was rapidly cooled (down to 25° C as fast as possible within ca. 30 min, from 25° C on at 8.6 K/h) down to 16 ° C. Solid purified BEMT (about 0.06 wt.-% with respect to the crude BEMT each) was added during cooling at 23 ° C, 22 ° C and 21 ° C as seeding crystals. After stirring for 3 h at 16 ° C, the suspension subsequently cooled down 0° C. After curing for 2 h, the suspension was filtered, and the residue was washed two times with acetone to yield purified BEMT in a yield of 98% with respect to the amount of crude BEMT. The particles of the crystallized BEMT were small (D_{50,3} = 5.8 µm) and therefore filtration and washing times were long (filter cake resistance 2 x 10¹³ mPas/m²). The amounts of side-product are listed in Table 1.

### Inventive Example 1 (IE1)

The crude BEMT melt obtained in RE1 was dissolved in acetone, the solution filtered and heated to 40° C to yield a 29 wt.-% solution of the crude BEMT in acetone. The mixture was rapidly (down to 25° C as fast as possible within ca. 30 min, from 25° C on at 8.6 K/h) cooled down to 16 ° C. Solid purified BEMT (about 0.06 wt.-% with respect to the crude BEMT each) was added during cooling at 23 ° C, 22 ° C and 21 ° C as seeding crystals. After stirring for 30 min at 16 ° C, the suspension was warmed to 30° C and subsequently cooled down to 25° C in a time period of 4.2 h (at a cooling speed of -1.2 K/h), and further cooled to 16 ° C in a time period of 3 h (at a cooling speed of -3 K/h). Finally, the suspension was cooled to 0° C in a time period of 3 h (at a cooling speed of -5.3 K/h). After curing for 2 h, the suspension was filtered, and the residue was washed two times with a sufficient amount of acetone to yield purified BEMT in a yield of 98 wt.-% with respect to the amount of crude BEMT). The particles of the crystallized BEMT were large (D_{50,3} = 11.3 µm) and therefore filtration and washing times were short (filter cake resistance 5.4 x 10¹² mPas/m²). In this example the fast filtration was employed for extended de-humidification by air-blowing which yielded a product with low amounts of soluble side-products (NK2 and NK5). The amounts of side-products are listed in Table 1.

### Inventive Example 2 (IE2)

To further reduce side-products, the crystallization step of IE1 can be repeated. Therefore, acetone-moist product obtained as in IE1 without the air-blowing step was dissolved in acetone and heated to 40° C to yield a 19.6 wt.-% solution of the product of IE1 in acetone. The mixture was rapidly (down to 25° C as fast as possible within ca. 30 min, from 25° C on at 8.6 K/h) cooled down to 16 ° C. Solid purified BEMT (about 0.06 wt.-% with respect to the weight of the product of IE1 each) was added during cooling at 23° C, 22 ° C and 21 ° C as seeding crystals. After stirring for 30 min at 16° C, the suspension was warmed to 32° C and subsequently cooled down to 25° C in the time period of 5.8 h (at a cooling rate of -1.2 K/h), and further cooled to 16 ° C in the time period of 3 h (at the cooling rate of -3 K/h). Finally, the suspension was cooled to 0 ° C in a time period of 3 h (at a cooling speed of -5.3 K/h). After curing for 2 h, the suspension was filtered, and the residue washed two times with a sufficient amount of acetone to yield purified BEMT in a yield of 98 wt.-% with respect to the amount t of crude BEMT). Filtration and washing step duration was comparable to IE1 (D_{50,3} = 12.0 µm; filter cake resistance 4.5 x 10¹² mPas/m²). In this case all side-products are significantly reduced compared to CE1. The amounts of side-products are listed in Table 1.

**Table 1: Results of the Examples RE1, CE1, and IE1-2**

| | Unit | RE1 | CE1 | IE1 | IE2 |
|---|---|---|---|---|---|
| NK1 - formula (I) | [wt.-%] | 0.4 | 0.08 | 0.05 | <0.02 |
| NK2 - formula (II) | [wt.-%] | 4.6 | 0.38 | <0.02 | <0.02 |
| NK3 - formula (III) | [wt.-%] | 0.2 | 0.11 | 0.10 | 0.04 |
| NK4 - formula (IV) | [wt.-%] | 1 | 0.49 | 0.40 | 0.11 |
| NK5 - formula (V) | [wt.-%] | 2.9 | 0.37 | 0.13 | <0.02 |
| NK6 - formula (VI) | [wt.-%] | 0.2 | 0.09 | 0.06 | <0.02 |
| NK7 - formula (VII) | [wt.-%] | 0.1 | <0.02 | <0.02 | <0.02 |
| NK8 - formula (VIII) | [wt.-%] | 0.2 | 0.18 | 0.16 | 0.14 |
| Median particle size D_{50,3} | [µm] | n.a. | 5.8 | 11.3 | 12.0 |
| Filter cake resistance | [mPas/m² ] | n.a. | 2E+13 | 5.4E+12 | 4.5E+12 |

| | | | | | |
|---|---|---|---|---|---|
| n.a.: Not available, as the crude product was obtained as a melt. | | | | | |

IE1 and IE2 showed a much-improved filter cake resistance as compared to CE1. The accelerated filtration and washing resulted mainly from the particles sizes that are significantly larger for IE1 and IE2 compared to CE1. Hence, the process of the present invention is faster and therefore less expensive. Additionally, the amounts of side-products contained in the final product were significantly reduced especially when comparing IE2 to CE1. A higher purity of BEMT is advantageous as it leads to healthier products and products having less impact on the environment.

## Claims

1. A process for increasing the content of 2,4-bis[4-(2-ethylhexyloxy)-2-hydroxyphenol]-6-(4-methoxyphenol)-1,3,5-triazine (BEMT) in a composition comprising BEMT, the process comprising the steps of:
a) providing a composition comprising BEMT and impurities;
b) dissolving said composition in a solvent mixture at a first dissolving temperature yielding an unsaturated BEMT solution, wherein the solvent mixture comprises at least one polar solvent;
c) decreasing the temperature of the unsaturated BEMT solution to a saturation temperature, wherein the saturation temperature is lower than the first dissolving temperature, yielding a saturated BEMT solution;
d) further decreasing the temperature of the saturated BEMT solution to a first crystallization temperature, wherein the first crystallization temperature is lower than the saturation temperature, thereby yielding a supersaturated BEMT solution;
e) adding seed crystals of BEMT in an amount in the range of from 0.00001 wt.-% to 20 wt.-% with respect to the total weight of said composition to the supersaturated BEMT solution during step d) yielding a first BEMT suspension comprising BEMT particles and the supersaturated BEMT solution;
f) heating the first BEMT suspension to a second dissolving temperature yielding a second BEMT suspension comprising less BEMT particles than the first BEMT suspension, wherein the second dissolving temperature is higher than the first crystallization temperature and lower than the first dissolving temperature;
g) decreasing the temperature of the second BEMT suspension to a second crystallization temperature yielding a final BEMT suspension comprising larger BEMT particles than the first BEMT suspension;
h) isolating the BEMT particles from the final BEMT suspension;
wherein the process preferably comprises one or more repetitions of steps b) to h) and wherein the second crystallization temperature is lower than the first crystallization temperature.

2. The process according to claim 1, wherein in step e), seed crystals of BEMT are added in two batches at two distinct times.

3. The process according to claim 1 or 2, wherein in step e) the temperature of the supersaturated BEMT solution is kept constant at the first crystallization temperature for at least 30 min.

4. The process according to any of the preceding claims, wherein in step g) the temperature of the second BEMT suspension is kept constant at the second crystallization temperature for at least 30 min.

5. The process according to any of the preceding claims, wherein the second dissolving temperature is higher than the maximum temperature the first BEMT suspension could reach by uptake of the crystallization enthalpy released in step g).

6. The process according to any one of the preceding claims, wherein the at least one polar solvent is selected from the group consisting of methanol, ethanol, 1-butanol, 2-butanol, isopropyl alcohol, acetone, methyl ethyl ketone, diethyl ketone, N,N-dimethyl formamide.

7. The process according to any one of the preceding claims, wherein step g) is carried out stepwise at two different cooling rates.

8. The process according to any one of the preceding claims, wherein in step d) the seed crystals are added at least once at a temperature in the range of 35° C to 20° C.

9. The process according to any one of the preceding claims, wherein step h) comprises a step of filtration and/or a step of washing.

10. A particulate composition comprising at least 98 wt.% 2,4-bis[4-(2-ethylhexyloxy)-2-hydroxyphenol]-6-(4-methoxyphenol)-1,3,5-triazine (BEMT), based on the total weight of the composition, and at least one of the compounds according to formulae (I) to (VIII): 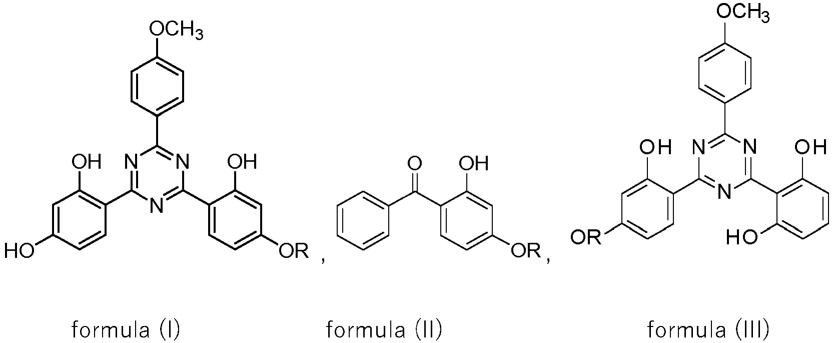 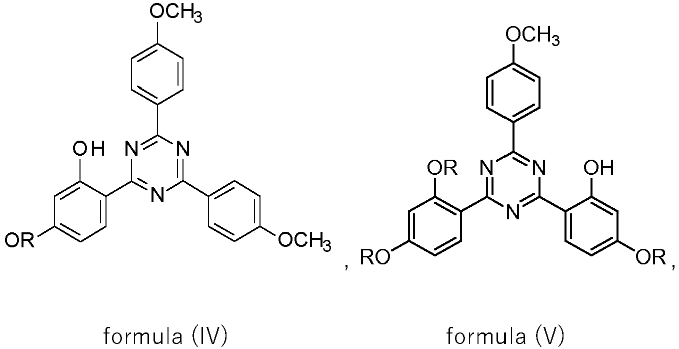 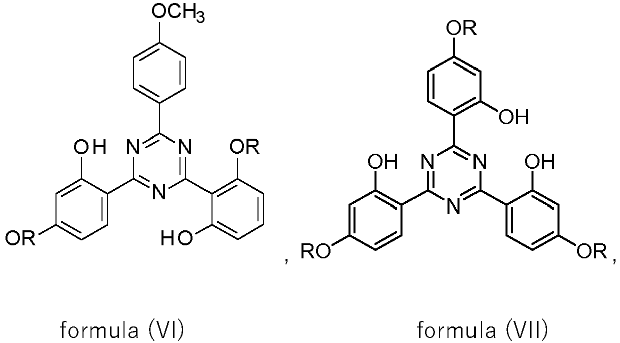 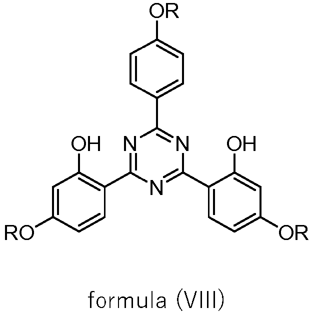 wherein
R is a 2-ethylhexyl residue;
the amount of the compound of formula (I) in the composition is less than 0.06 wt.-%, based on the total weight of the composition;
the amount of the compound of formula (II) in the composition is less than 0.11 wt.-%, based on the total weight of the composition;
the amount of the compound of formula (III) in the composition is less than 0.11 wt.- %, based on the total weight of the composition;
the amount of the compound of formula (IV) in the composition is less than 0.41 wt.- %, based on the total weight of the composition;
the amount of the compound of formula (V) in the composition is less than 0.14 wt.-%, based on the total weight of the composition;
the amount of the compound of formula (VI) in the composition is less than 0.07 wt.- %, based on the total weight of the composition;
the amount of the compound of formula (VII) in the composition is less than 0.05 wt.- %, based on the total weight of the composition;
the amount of the compound of formula (VIII) in the composition is less than 0.17 wt.- %, based on the total weight of the composition; wherein the particulate composition has a median particle size D_{50,3} of more than 10 µm, determined according to ISO 13320:2020.

11. The particulate composition according to claim 10, wherein the amount of BEMT in the composition is more than 98 wt.-% with respect to the total weight of the composition.

12. The particulate composition according to claim 10, wherein the amount of BEMT in the composition is more than 99 wt.-% with respect to the total weight of the composition.

13. Use of the particulate composition according to any one of claims 10 to 12 for protection of human and animal hair and skin from the damaging effects of UV radiation.

14. A cosmetic preparation comprising the particulate composition according to any one of claims 10 to 12 with cosmetically compatible carriers and auxiliaries.

15. The cosmetic preparation according to claim 14 further comprising other UV protection substances.

## Patentansprüche

1. Verfahren zur Erhöhung des Gehalts an 2,4-bis[4-(2-ethylhexyloxy)-2-hydroxyphenol]-6-(4-methoxyphenol)-1,3,5-triazin (BEMT) in einer Zusammensetzung, die BEMT umfasst, wobei das Verfahren die folgenden Schritte umfasst:
a) Bereitstellen einer Zusammensetzung, die BEMT und Verunreinigungen umfasst;
b) Lösen der genannten Zusammensetzung in einem Lösungsmittelgemisch bei einer ersten Lösetemperatur, wodurch eine ungesättigte BEMT-Lösung erhalten wird, wobei das Lösungsmittelgemisch mindestens ein polares Lösungsmittel umfasst;
c) Absenken der Temperatur der ungesättigten BEMT-Lösung auf eine Sättigungstemperatur, wobei die Sättigungstemperatur niedriger ist als die erste Lösetemperatur, wodurch eine gesättigte BEMT-Lösung erhalten wird;
d) weiteres Absenken der Temperatur der gesättigten BEMT-Lösung auf eine erste Kristallisationstemperatur, wobei die erste Kristallisationstemperatur niedriger ist als die Sättigungstemperatur, wodurch eine übersättigte BEMT-Lösung erhalten wird;
e) Zusetzen von Impfkristallen von BEMT in einer Menge im Bereich von 0,00001 Gew.-% bis 20 Gew.-%, bezogen auf das Gesamtgewicht der genannten Zusammensetzung, zu der übersättigten BEMT-Lösung während Schritt d), wodurch eine erste BEMT-Suspension erhalten wird, die BEMT-Partikel und die übersättigte BEMT-Lösung umfasst;
f) Erwärmen der ersten BEMT-Suspension auf eine zweite Lösetemperatur, wodurch eine zweite BEMT-Suspension erhalten wird, die weniger BEMT-Partikel als die erste BEMT-Suspension umfasst, wobei die zweite Lösetemperatur höher ist als die erste Kristallisationstemperatur und niedriger als die erste Lösetemperatur;
g) Absenken der Temperatur der zweiten BEMT-Suspension auf eine zweite Kristallisationstemperatur, wodurch eine finale BEMT-Suspension erhalten wird, die größere BEMT-Partikel als die erste BEMT-Suspension umfasst;
h) Abtrennen der BEMT-Partikel aus der finalen BEMT-Suspension;
wobei das Verfahren vorzugsweise eine oder mehrere Wiederholungen der Schritte b) bis h) umfasst und wobei die zweite Kristallisationstemperatur niedriger ist als die erste Kristallisationstemperatur.

2. Verfahren nach Anspruch 1, wobei in Schritt e) Impfkristalle von BEMT in zwei Chargen zu zwei unterschiedlichen Zeitpunkten zugesetzt werden.

3. Verfahren nach Anspruch 1 oder 2, wobei in Schritt e) die Temperatur der übersättigten BEMT-Lösung für mindestens 30 min konstant bei der ersten Kristallisationstemperatur gehalten wird.

4. Verfahren nach einem der vorhergehenden Ansprüche, wobei in Schritt g) die Temperatur der zweiten BEMT-Suspension für mindestens 30 min konstant bei der zweiten Kristallisationstemperatur gehalten wird.

5. Verfahren nach einem der vorhergehenden Ansprüche, wobei die zweite Lösetemperatur höher ist als die maximale Temperatur, die die erste BEMT-Suspension durch Aufnahme der in Schritt g) freigesetzten Kristallisationsenthalpie erreichen könnte.

6. Verfahren nach einem der vorhergehenden Ansprüche, wobei das mindestens eine polare Lösungsmittel aus der Gruppe bestehend aus Methanol, Ethanol, 1-Butanol, 2-Butanol, Isopropylalkohol, Aceton, Methyl-Ethyl-Keton, Diethylketon, N,N-Dimethylformamid ausgewählt ist.

7. Verfahren nach einem der vorhergehenden Ansprüche, wobei Schritt g) stufenweise bei zwei unterschiedlichen Abkühlraten durchgeführt wird.

8. Verfahren nach einem der vorhergehenden Ansprüche, wobei in Schritt d) die Impfkristalle mindestens einmal bei einer Temperatur im Bereich von 35 °C bis 20 °C zugesetzt werden.

9. Verfahren nach einem der vorhergehenden Ansprüche, wobei Schritt h) einen Filtrationsschritt und/oder einen Waschschritt umfasst.

10. Partikuläre Zusammensetzung, umfassend mindestens 98 Gew.-% 2,4-bis[4-(2-ethylhexyloxy)-2-hydroxyphenol]-6-(4-methoxyphenol)-1,3,5-triazin (BEMT), bezogen auf das Gesamtgewicht der Zusammensetzung, und mindestens eine der Verbindungen gemäß den Formeln (I) bis (VIII): 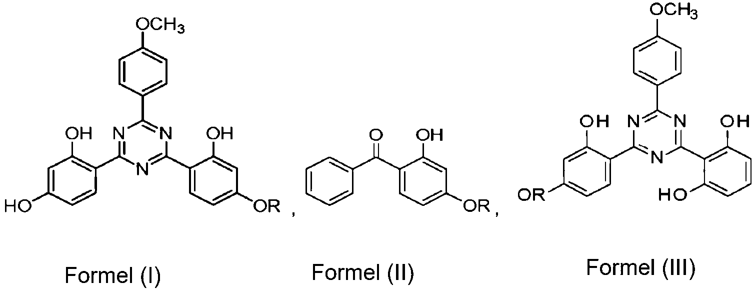 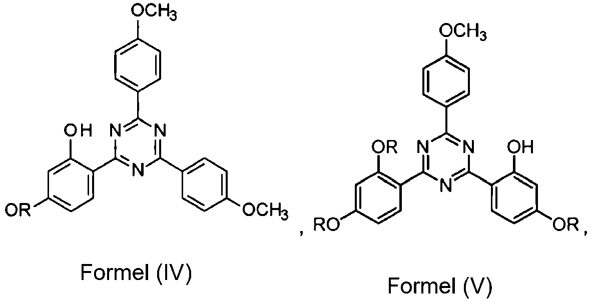 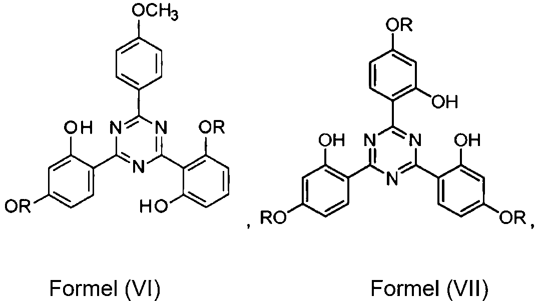 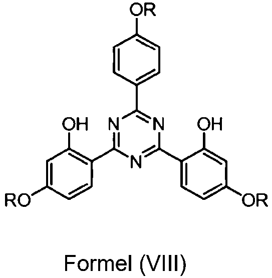 wobei
R ein 2-Ethylhexylrest ist;
die Menge der Verbindung der Formel (I) in der Zusammensetzung weniger als 0,06 Gew.-% beträgt, bezogen auf das Gesamtgewicht der Zusammensetzung;
die Menge der Verbindung der Formel (II) in der Zusammensetzung weniger als 0,11 Gew.-% beträgt, bezogen auf das Gesamtgewicht der Zusammensetzung;
die Menge der Verbindung der Formel (III) in der Zusammensetzung weniger als 0,11 Gew.-% beträgt, bezogen auf das Gesamtgewicht der Zusammensetzung;
die Menge der Verbindung der Formel (IV) in der Zusammensetzung weniger als 0,41 Gew.-% beträgt, bezogen auf das Gesamtgewicht der Zusammensetzung;
die Menge der Verbindung der Formel (V) in der Zusammensetzung weniger als 0,14 Gew.-% beträgt, bezogen auf das Gesamtgewicht der Zusammensetzung;
die Menge der Verbindung der Formel (VI) in der Zusammensetzung weniger als 0,07 Gew.-% beträgt, bezogen auf das Gesamtgewicht der Zusammensetzung;
die Menge der Verbindung der Formel (VII) in der Zusammensetzung weniger als 0,05 Gew.-% beträgt, bezogen auf das Gesamtgewicht der Zusammensetzung;
die Menge der Verbindung der Formel (VIII) in der Zusammensetzung weniger als 0,17 Gew.-% beträgt, bezogen auf das Gesamtgewicht der Zusammensetzung;
wobei die partikuläre Zusammensetzung eine Medianpartikelgröße D_{50,3} von mehr als 10 µm aufweist, bestimmt gemäß ISO 13320:2020.

11. Partikuläre Zusammensetzung nach Anspruch 10, wobei die Menge an BEMT in der Zusammensetzung mehr als 98 Gew.-% bezogen auf das Gesamtgewicht der Zusammensetzung beträgt.

12. Partikuläre Zusammensetzung nach Anspruch 10, wobei die Menge an BEMT in der Zusammensetzung mehr als 99 Gew.-% bezogen auf das Gesamtgewicht der Zusammensetzung beträgt.

13. Verwendung der partikulären Zusammensetzung nach einem der Ansprüche 10 bis 12 zum Schutz von menschlichem und tierischem Haar und Haut vor den schädigenden Wirkungen der UV-Strahlung.

14. Kosmetische Zubereitung, umfassend die partikuläre Zusammensetzung nach einem der Ansprüche 10 bis 12 mit kosmetisch verträglichen Träger- und Hilfsstoffen.

15. Kosmetische Zubereitung nach Anspruch 14, ferner umfassend andere UV-Schutzstoffe.

## Revendications

1. Un procédé visant à augmenter la teneur en 2,4-bis[4-(2-éthylhexyloxy)-2-hydroxyphénol]-6-(4-méthoxyphénol)-1,3,5-triazine (BEMT) dans une composition comprenant du BEMT, le procédé comprenant les étapes consistant à:
a) fournir une composition comprenant du BEMT et des impuretés;
b) dissoudre ladite composition dans un mélange de solvants à une première température de dissolution, ce qui donne une solution de BEMT non saturée, dans laquelle le mélange de solvants comprend au moins un solvant polaire;
c) abaisser la température de la solution de BEMT non saturée à une température de saturation, dans laquelle la température de saturation est inférieure à la première température de dissolution, ce qui donne une solution de BEMT saturée;
d) abaisser encore la température de la solution de BEMT saturée à une première température de cristallisation, dans laquelle la première température de cristallisation est inférieure à la température de saturation, de manière à obtenir une solution de BEMT sursaturée;
e) ajouter des cristaux germes de BEMT en une quantité comprise dans l'intervalle de 0,00001 % en poids à 20 % en poids par rapport au poids total de ladite composition à la solution de BEMT sursaturée pendant l'étape d), ce qui donne une première suspension de BEMT comprenant des particules de BEMT et la solution de BEMT sursaturée;
f) chauffer la première suspension de BEMT à une deuxième température de dissolution, ce qui donne une deuxième suspension de BEMT comprenant moins de particules de BEMT que la première suspension de BEMT, dans laquelle la deuxième température de dissolution est supérieure à la première température de cristallisation et inférieure à la première température de dissolution;
g) abaisser la température de la deuxième suspension de BEMT à une deuxième température de cristallisation, ce qui donne une suspension finale de BEMT comprenant des particules de BEMT plus grandes que la première suspension de BEMT;
h) isoler les particules de BEMT de la suspension finale de BEMT;
dans lequel le procédé comprend de préférence une ou plusieurs répétitions des étapes b) à h) et dans lequel la deuxième température de cristallisation est inférieure à la première température de cristallisation.

2. Le procédé selon la revendication 1, dans lequel, à l'étape e), des cristaux germes de BEMT sont ajoutés en deux lots à deux moments distincts.

3. Le procédé selon la revendication 1 ou 2, dans lequel, à l'étape e), la température de la solution de BEMT sursaturée est maintenue constante à la première température de cristallisation pendant au moins 30 min.

4. Le procédé selon l'une quelconque des revendications précédentes, dans lequel, à l'étape g), la température de la deuxième suspension de BEMT est maintenue constante à la deuxième température de cristallisation pendant au moins 30 min.

5. Le procédé selon l'une quelconque des revendications précédentes, dans lequel la deuxième température de dissolution est supérieure à la température maximale que la première suspension de BEMT pourrait atteindre par absorption de l'enthalpie de cristallisation libérée à l'étape g).

6. Le procédé selon l'une quelconque des revendications précédentes, dans lequel ledit au moins un solvant polaire est choisi dans le groupe constitué de méthanol, éthanol, 1-butanol, 2-butanol, alcool isopropylique, acétone, méthyléthylcétone, diéthylcétone, N,N-diméthylformamide.

7. Le procédé selon l'une quelconque des revendications précédentes, dans lequel l'étape g) est réalisée de manière échelonnée à deux vitesses de refroidissement différentes.

8. Le procédé selon l'une quelconque des revendications précédentes, dans lequel, à l'étape d), les cristaux germes sont ajoutés au moins une fois à une température comprise dans l'intervalle de 35 °C à 20 °C.

9. Le procédé selon l'une quelconque des revendications précédentes, dans lequel l'étape h) comprend une étape de filtration et/ou une étape de lavage.

10. Une composition particulaire comprenant au moins 98 % en poids de 2,4-bis[4-(2-éthylhexyloxy)-2-hydroxyphénol]-6-(4-méthoxyphénol)-1,3,5-triazine (BEMT), sur la base du poids total de la composition, et au moins un des composés selon les formules (I) à (VIII): 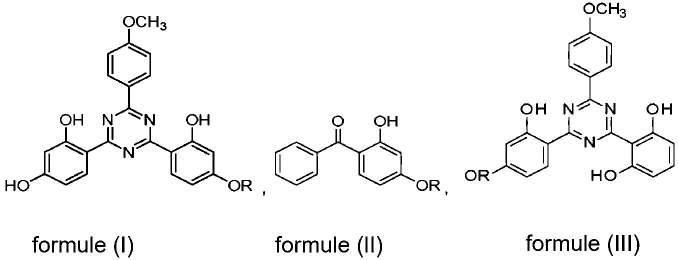 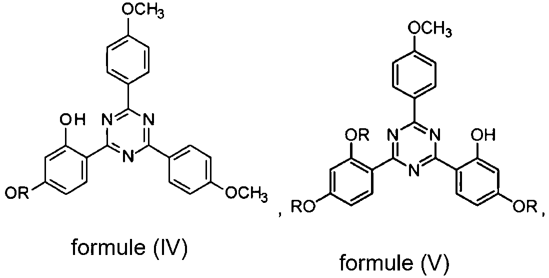 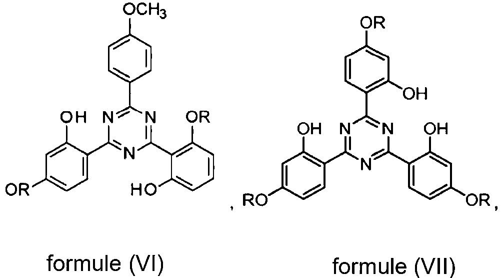 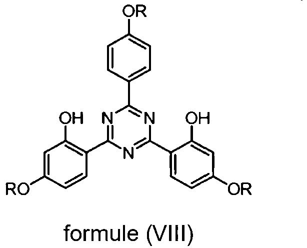 dans lequel
R est un résidu 2-éthylhexyle;
la quantité du composé de la formule (I) dans la composition est inférieure à 0,06 % en poids, sur la base du poids total de la composition;
la quantité du composé de la formule (II) dans la composition est inférieure à 0,11 % en poids, sur la base du poids total de la composition;
la quantité du composé de la formule (III) dans la composition est inférieure à 0,11 % en poids, sur la base du poids total de la composition;
la quantité du composé de la formule (IV) dans la composition est inférieure à 0,41 % en poids, sur la base du poids total de la composition;
la quantité du composé de la formule (V) dans la composition est inférieure à 0,14 % en poids, sur la base du poids total de la composition;
la quantité du composé de la formule (VI) dans la composition est inférieure à 0,07 % en poids, sur la base du poids total de la composition;
la quantité du composé de la formule (VII) dans la composition est inférieure à 0,05 % en poids, sur la base du poids total de la composition;
la quantité du composé de la formule (VIII) dans la composition est inférieure à 0,17 % en poids, sur la base du poids total de la composition;
dans lequel la composition particulaire présente une taille de particules médiane D_{50,3} supérieure à 10 µm, déterminée selon ISO 13320:2020.

11. La composition particulaire selon la revendication 10, dans laquelle la quantité de BEMT dans la composition est supérieure à 98 % en poids par rapport au poids total de la composition.

12. La composition particulaire selon la revendication 10, dans laquelle la quantité de BEMT dans la composition est supérieure à 99 % en poids par rapport au poids total de la composition.

13. Utilisation de la composition particulaire selon l'une quelconque des revendications 10 à 12 pour la protection des cheveux et de la peau humains et animaux contre les effets dommageables du rayonnement UV.

14. Une préparation cosmétique comprenant la composition particulaire selon l'une quelconque des revendications 10 à 12 avec des supports et des auxiliaires compatibles sur le plan cosmétique.

15. La préparation cosmétique selon la revendication 14 comprenant en outre d'autres substances de protection UV.
